# EUROPÄISCHE PATENTSCHRIFT

(11) **EP 2 509 511 B1**
(45) Veröffentlichungstag und Bekanntmachung des Hinweises auf die Patenterteilung: **26.08.2015**
(21) Anmeldenummer: 10785411.9
(22) Anmeldetag: 30.11.2010
(51) Int. Cl.: A61B 17/00, A61B 19/00, A61B 17/29

(54) **CHIRURGISCHES MANIPULATIONSINSTRUMENT**
SURGICAL MANIPULATION INSTRUMENT
INSTRUMENT DE MANIPULATION À USAGE CHIRURGICAL

(30) Priorität: 07.12.2009 DE 102009056982
(43) Veröffentlichungstag der Anmeldung: 17.10.2012
(73) Patentinhaber: Deutsches Zentrum für Luft- und Raumfahrt e.V., 51147 Köln (DE)
(72) Erfinder: SEIBOLD, Ulrich, Burnaby BC V3N 4Z4 (CA); LANTERMANN, Sophie, 81679 München (DE); STROHMAYR, Michael, 81667 München (DE)
(74) Vertreter: Von Kreisler Selting Werner - Partnerschaft von Patentanwälten und Rechtsanwälten mbB
(86) Internationale Anmeldenummer: PCT/EP2010/068505
(87) Internationale Veröffentlichungsnummer: WO 2011/069862

(56) Entgegenhaltungen:
- EP-A1- 0 598 202
- WO-A1-2009/102102
- US-A1- 2008 177 283
- US-A1- 2008 249 551

## Beschreibung

Die Erfindung bezieht sich auf ein chirurgisches Manipulationsinstrument, mit einer Kupplung, die ein extrakorporales Antriebsteil mit einem teilweise intrakorporalen Manipulatorteil trennbar verbindet.

Chirurgische Instrumente, die nicht zum einmaligen Gebrauch, sondern zum mehrfachen Gebrauch konzipiert sind, müssen nach jedem Gebrauch sterilisiert werden. Die Sterilisierung kann mit Hilfe nicht-thermischer oder thermischer Verfahren durchgeführt werden. Im klinischen Alltag wird im Wesentlichen mit thermischen Verfahren sterilisiert, insbesondere durch die sogenannte Autoklavierung. Bei der Autoklavierung wird das zu sterilisierende Instrument über einen bestimmten Zeitraum überspanntem Wasserdampf ausgesetzt, der alle zu sterilisierenden Oberflächen benetzen muss. Das zu sterilisierende Instrument ist dabei im Autoklaven Temperaturen von bis zu 156 °C und Drücken bis zu 2 bar über eine Dauer von bis zu 40 Min. ausgesetzt. Die Autoklavierung muss nach jedem Gebrauch des Instruments wiederholt werden, so dass im Laufe eines Instrumentenlebens bis zu mehrere Hundert Autoklavierungen durchlaufen werden können.

Seit den 1980er Jahren gewinnt die sogenannte minimal invasive Chirurgie immer mehr an Bedeutung. Hierbei werden lange, schlanke Manipulationsinstrumente durch nur kleine Öffnungen in der Oberhaut vorgeschoben. Das intrakorporale Operationsfeld wird mit Hilfe einer auf die gleiche Weise eingebrachten stabförmigen Kamera und eines extrakorporalen Monitors beobachtet. Die minimal invasive Chirurgie bietet insbesondere Vorteile für den Patienten, nämlich geringe Traumatisierung, kurze Rekonvaleszenzzeiten, geringere postoperative Schmerzen, geringeren Blutverlust, geringeres Infektionsrisiko, geringeres Risiko für Wundheilungsstörungen, bessere kosmetische Ergebnisse etc. Nachteile der minimal invasiven Chirurgie sind u.a. die eingeschränkte Bewegungsfreiheit der chirurgischen Instrumente. Durch die als feststehend anzusehende Durchtrittsstelle durch das Oberhaut- und Fettgewebe, die einen invarianten Punkt bildet, ergeben sich umgekehrte Bewegungsverhältnisse bzw. eine gestörte Hand-Auge-Koordination zum Monitorbild. Zwei Freiheitsgrade der Bewegung sind durch den invarianten Punkt gebunden, d.h. nicht jeder Punkt im Arbeitsraum kann mit beliebiger Orientierung des funktionalen Instrumentenendes erreicht werden.

Minimal invasive Manipulationsinstrumente, die intrakorporal zusätzliche Freiheitsgrade der Bewegung bieten, können zu einer erhöhten intrakorporalen Manipulabilität verhelfen und stellen damit eine wichtige Verbesserung für die minimal invasive Chirurgie dar. Die zusätzlichen Freiheitsgrade müssen zielgerichtet bewegt werden. Dies ist mit manueller Bedienung zwar möglich, jedoch erfordert dies großes Geschick und Übung. Ein robotergestützter telemanipulierter Ansatz, bei dem der Chirurg abseits des Patienten an einer ergonomisch geformten Konsole sitzt und mit Hilfe einer geeigneten Mensch-Maschine-Schnittstelle das chirurgische Manipulationsinstrument mit Hilfe des Monitors führt, ohne über die Kinematik und deren Aktuierung nachdenken zu müssen, ist daher sinnvoll. Das chirurgische Manipulationsinstrument wird dabei rechnergestützt aktuiert und führt die vom Chirurgen gewünschte Bewegung entsprechend aus.

Die Aktuatoren zum Antrieb des chirurgischen Manipulationsinstruments können in der Regel jedoch nicht autoklaviert werden. Daher ist eine Trennbarkeit des extrakorporalen Antriebsteiles von dem teilweise intrakorporalen Manipulatorteil erforderlich.

Das chirurgische Manipulationsinstrument wird daher zweiteilig und durch eine Kupplung trennbar in ein extrakorporales Antriebsteil und ein teilweise intrakorporales Manipulatorteil getrennt.

Aus US 6 491 707 A1 ist ein chirurgisches Manipulationsinstrument mit einer Kupplung zur Trennung des Antriebsteiles von dem Manipulatorteil bekannt. Hierbei werden durch die Antriebseinrichtung Betätigungselemente wie Wellen rotiert und über die Kupplungseinrichtung die Rotations- und Drehbewegung an Betätigungselemente des Manipulatorteils übertragen. Durch diese zweiten Betätigungselemente wird der Endeffektor des Manipulatorteils betätigt. Die Kupplungsteile weisen jeweils drehbare Kupplungskörper auf, die axiale Stifte bzw. Bohrungen aufweisen und axial miteinander verkuppelt bzw. voneinander getrennt werden. Da die drehbaren einander gegenüberstehenden Kupplungskörper nicht zusammen kuppelbar sind, wenn sie nicht genau zueinander ausgerichtet sind, muss beim Einkuppeln ein Suchlauf für alle Kupplungskörper-Paare durchgeführt werden. Die Kupplungskörper rotieren jeweils so lange bis eine Position gefunden ist, in der alle Kupplungskörper-Paare in einer zusammenführbaren Kupplungsposition stehen. Eine ähnliche Kupplung ist aus US 2001 003 1983 A1 bekannt. Die Kupplungsteile weisen hier halbzylindrisch geformte Kupplungskörper auf. Für einen leichtgängigen Kupplungsvorgang ist es erforderlich, ein gewisses Mindest-Spiel zwischen den Kupplungskörpern im zusammengekuppelten Zustand vorzusehen. Dieses Spiel wirkt sich jedoch beim Betrieb des Manipulatorinstruments nachteilig aus bzw. macht eine automatische Regelung ggf. sogar unmöglich.

In WO 2009/102102 A1 wird ein Kopplungselement eines chirurgischen Manipulationsinstruments offenbart. Dieses besteht aus mehreren nebeneinander angeordneten scheibenförmigen Antriebsrädern in einem extrakorporalen Antriebsteil und ebenso vielen nebeneinander angeordneten scheibenförmigen Antriebsrädern in einem teilweise intrakorporalen Manipulatorteil. Dabei sind Antriebsteil und Manipulatorteil über eine Kupplungseinrichtung derart zueinander angeordnet, dass ein Antriebsrad des Antriebsteils das entsprechende Antriebsrad des Manipulatorteils in Bewegung versetzt. Diese Drehbewegung kann dann in eine axiale Bewegung umgesetzt werden und somit für die Bewegung des Endeffektors des Manipulationsinstruments verwendet werden.

Ferner wird in EP 0 598 202 A1 ein Verbindungsselement beschrieben, das einen Manipulatorteil mit einem Antriebsteil, der einen manuellen Aktuator beinhaltet, fest miteinander verbindet. Die Aktuierung durch den Chirurgen erfolgt in Form einer Drehbewegung des Aktuators. Diese Drehbewegung wird über ein Gestänge in eine axiale Bewegung übertragen. Der Manipulatorteil ist dabei mit dem Antriebsteil durch einen Stift, der als Achse dient, fest verbunden.

Aufgabe der Erfindung ist es ein chirurgisches Manipulationsinstrument mit sowohl antriebsseitig als auch manipulatorseitig axial verschiebbaren Elementen zu schaffen, wobei die Betätigungselemente durch eine zuverlässige Kupplungseinrichtung miteinander verbunden sind, wobei eine Vergrößerung oder Verkleinerung der Bewegung realisiert werden kann.

Die Lösung der Aufgabe erfolgt erfindungsgemäß durch die Merkmale des Anspruchs 1.

Durch neuentwickelte axiale Bewegungen erzeugende Antriebseinrichtungen ist es möglich erste mit der Antriebseinrichtung verbundene Betätigungselemente wie Seile oder Stäbe nicht zu drehen sondern axial, d.h. in der Linksrichtung zu verschieben. Auch auf der Seite des Manipulatorteils ist es wünschenswert ebenfalls axial verschiebbare zweite Betätigungselemente zum Betätigen des Endeffektors vorzusehen. Die lineare Betätigung des Endeffektors ist hinsichtlich der Handhabbarkeit vorteilhaft.

Das erfindungsgemäße chirurgische Manipulationsinstrument, das insbesondere für die minimal invasive Chirurgie geeignet ist, weist eine extrakorporale Antriebseinrichtung sowie einen teilweise intrakorporal anordnenbaren Manipulatorteil auf. Die Antriebseinrichtung weist mehrere axial verschiebbare erste Betätigungselemente auf. Bei den Betätigungselementen handelt es sich beispielsweise um Stangen oder Seile, durch die Axialkräfte übertragen werden. Das Manipulatorteil weist ebenfalls mehrere axial verschiebbare zweite Betätigungselemente auf, bei denen es sich beispielsweise ebenfalls um Stangen und/oder Seile handelt. Die Betätigungselemente dienen zur Betätigung eines Endeffektors.

Die ersten Betätigungselemente sind mit den zweiten Betätigungselementen über eine Kupplungseinrichtung miteinander verbunden. Über die Kupplungseinrichtung erfolgt eine lösbare Verbindung von Betätigungselementen-Paaren. Die Kupplungseinrichtung weist ein erstes Kupplungselement auf, das mit dem ersten Betätigungselement verbunden ist. Ferner weist die Kupplungseinrichtung ein zweites Kupplungselement auf, das mit dem zweiten Betätigungselement verbunden ist. Hierbei sind vorzugsweise mindestens zwei über entsprechende Kupplungseinrichtungen miteinander verbundene Betätigungselemente vorgesehen, so dass ein erstes und ein zweites Betätigungselement jeweils ein Betätigungselemente-Paar bilden. Erfindungsgemäß ist zwischen dem zweiten Kupplungselement und dem zweiten Betätigungselement ein schwenkbares Zwischenelement vorgesehen. Selbstverständlich kann ein entsprechendes schwenkbares Zwischenelement auch zwischen dem ersten Kupplungselement und dem ersten Betätigungselement vorgesehen sein, wobei im nachfolgenden die Erfindung anhand eines zwischen dem zweiten Kupplungselement und dem zweiten Betätigungselement vorgesehenen schwenkbaren Zwischenelements beschrieben wird. Das schwenkbare Zwischenelement dient dazu, eine axiale Bewegung des ersten Betätigungselements in eine Schwenkbewegung zu überführen. Die Schwenkbewegung des Zwischenelements führt sodann zu einer axialen Bewegung des zweiten Betätigungselements. Hierbei ist es beispielsweise je nach Ausgestaltung des schwenkbaren Zwischenelements möglich eine Vergrößerung oder Verkleinerung der Bewegung des ersten Betätigungselements zu realisieren. Insbesondere ist es möglich, eine Bewegung des ersten Betätigungselements derart zu verringern, dass sich das zweite Betätigungselement um eine geringere Wegstrecke bewegt. Hierdurch ist ein sehr präzises Handling möglich.

In bevorzugter Weiterbildung der Erfindung weist das zweite Kupplungselement einen Schwenkarm auf. Der Schwenkarm ist vorzugsweise einstückig mit dem Zwischenelement verbunden. Bei dieser Ausführungsform ist es bevorzugt, dass der Schwenkarm über das erste Kupplungselement mit dem ersten Betätigungselement verbunden ist, wobei hier insbesondere eine starre Verbindung erfolgt. Durch axiales Verschieben des ersten Betätigungselements erfolgt somit ein Schwenken bzw. Verdrehen des Schwenkarms um die Achse des schwenkbaren Zwischenelements.

Zur Verbindung des Schwenkarms mit dem ersten Kupplungselement weist der Schwenkarm vorzugsweise eine gabelförmige Ausnehmung auf. In diese gabelförmige Ausnehmung greift ein Ansatz des ersten Kupplungselements ein. Hierbei kann der Ansatz einen beispielsweise kreiszylindrischen Querschnitt aufweisen. Die gabelförmige Ausnehmung ist sodann vorzugsweise schlitzförmig ausgebildet und weist einen abgerundeten Endbereich auf, so dass der Ansatz möglichst flächig in diesem Bereich an der Ausnehmung anliegt. Die Ausnehmung und der Ansatz sind vorzugsweise in der Außengestaltung aufeinander angepasst, um eine möglichst große Anlagefläche zu realisieren. Auch kann der Ansatz einen quaderförmigen, elliptischen oder auch ovalen Querschnitt aufweisen. Die Ausnehmung ist vorzugsweise wiederum entsprechend ausgebildet, um eine möglichst große Anlagefläche zu realisieren.

Da vorzugsweise zwei, insbesondere drei Betätigungselemente-Paare vorgesehen sind, ist es bevorzugt, dass die Verbindung der Kuppelelemente gemeinsam erfolgt. Dies kann in bevorzugter Ausführungsform derart erfolgen, dass die Ansätze der ersten Kupplungselemente derart angeordnet sind, dass durch ein gemeinsames Drehen der Kupplungselemente um ihre gemeinsame Schwenkachse ein Eingreifen in die gabelförmigen Ausnehmungen erfolgt. Die gabelförmigen Ausnehmungen sind hierbei entsprechend auf einer Kreislinie angeordnet, so dass die Ansätze in die Ausnehmungen hineingedreht werden können. Ebenso ist es möglich, dass die gabelförmigen Ausnehmungen der zweiten Kupplungselemente in die gleiche Richtung offen sind, so dass entsprechend ausgerichtete Ansätze der ersten Kupplungselemente durch seitliches Verschieben gleichzeitig in alle gabelförmigen Ausnehmungen eingeschoben werden können.

Vorzugsweise ist die Schwenkachse des Zwischenelements im Wesentlichen senkrecht zu der Bewegungsrichtung, zumindest eines insbesondere beider Betätigungselemente. Hierbei sind die Betätigungselemente vorzugsweise derart ausgerichtet, dass die Längsachsen der Betätigungselemente zueinander parallel, insbesondere koaxial sind.

In bevorzugter Weiterbildung der Erfindung weist das Zwischenelement ein zumindest teilkreisförmiges Scheibenelement, insbesondere ein als vollständiger Kreis ausgebildetes Scheibenelement auf. An einem Außenumfang des Scheibenelements ist vorzugsweise eine Rille vorgesehen, in der insbesondere ein seilförmig ausgebildetes Verbindungselement angeordnet ist. Ein Schwenken des Zwischenelements, insbesondere durch Verschieben des Schwenkarms in tangentiale Richtung bewirkt somit ein Drehen bzw. Schwenken des Scheibenelements. Hierdurch wird je nach Schwenkrichtung und Anordnung des seilförmigen Verbindungselements ein Auf- bzw. Abwickeln des Verbindungselements bewirkt. Beispielsweise durch ein Aufwickeln des Verbindungselements um einen durchaus geringen Winkel, erfolgt ein Übertragen einer axialen Zugkraft auf die zweite Betätigungseinrichtung. Das Verbindungselement ist somit jeweils mit einem zweiten Betätigungselement verbunden oder geht unmittelbar in das Betätigungselement über. Um Axialkräfte in beide Richtungen übertragen zu können, ist es möglich das zumindest teilkreisförmig ausgebildete Scheibenelement mit zwei an dessen Außenumfang vorgesehenen Verbindungselementen, die insbesondere in einer Rille angeordnet sind, zu verbinden. Hierbei sind die beiden Verbindungselemente in unterschiedliche Richtungen auf das Scheibenelement aufgewickelt bzw. umgeben das Scheibenelement in unterschiedliche Richtungen. Es ist ausreichend, dass das Verbindungselement das Scheibenelement um einen gewissen Winkel, beispielsweise 90 Grad, umgibt. Durch ein entsprechendes Drehen des Scheibenelements erfolgt somit jeweils ein Lösen eines der beiden Verbindungselemente und ein Ziehen an dem jeweils anderen Verbindungselement.

Bei einer weiteren bevorzugten Ausführungsform weist das schwenkbare Zwischenelement einen vorzugsweise starr mit dem Zwischenelement verbundenen Verbindungsarm auf. Der Verbindungsarm ist vorzugsweise in einem Winkel von ungleich 0 Grad zu dem Schwenkarm angeordnet. Somit erfolgt eine Umsetzung einer Linearbewegung des zweiten Betätigungselements, in bevorzugter Ausführungsform in ein Verschwenken des Schwenkarms. Dies führt sodann zu einem entsprechenden Verschwenken des Verbindungsarms. Der Verbindungsarm kann sodann unmittelbar mit dem zweiten Betätigungselement oder über weitere Zwischenelemente mit diesem verbunden sein. Durch das Vorsehen unterschiedlicher Armlängen und/oder weiterer Zwischenelemente kann eine Über- oder Untersetzung der Bewegung des ersten Betätigungselements realisiert werden. Vorzugsweise erfolgt eine Verbindung zwischen dem Verbindungsarm und dem zweiten Betätigungselement über elastische, insbesondere seilförmige Verbindungselemente. Diese sind in bevorzugter Ausführungsform über eine oder mehrere Umlenkrollen geführt.

Gemäß einer weiteren Ausführungsform eines chirurgischen Manipulationsinstruments, bei dem eine Bewegung der ersten und zweiten Betätigungselemente in axiale Richtung erfolgt, ist dies im wesentlichen durch Verschrauben eines Kupplungselementes durchgeführt. Hierbei sind in bevorzugter Ausführungsform die Befestigungselemente-Paare derart ausgebildet, dass eines der beiden Befestigungselemente ein Außengewinde und das andere einen Ansatz mit einem Innengewinde aufweist. Um die einzelnen Befestigungselemente nicht gesondert voneinander verschrauben zu müssen, ist in einer weiteren bevorzugten Ausführungsform ein Ring vorgesehen, der die Befestigungselemente im Bereich der Kupplung umgibt. Ein Teil der Befestigungselemente, bevorzugt solche mit der Innenverzahnung, ist in dem Betätigungselement drehbar gelagert, damit kein Verdrehen der Betätigungselemente nötig ist. Des Weiteren ist zwischen den Befestigungselementen ein elastisches Bauteil vorgesehen, welches Festigungstoleranzen ausgleicht und ein gleichzeitiges Festschrauben aller Befestigungselemente ermöglicht. Der Ring weist eine Innenverzahnung auf, die in Verzahnungen eingreift, die am Außenumfang der Befestigungselemente vorgesehen sind. Durch Drehen dieses Rings erfolgt somit ein Drehen der einzelnen Betätigungselemente um ihre Längsachse. Hierdurch erfolgt ein Verschrauben der Betätigungselemente-Paare. Gegebenenfalls kann der Ring von einem weiteren Ring angetrieben werden , wobei zwischen den beiden Ringen mindestens ein Zahnrad ähnlich eines Planetenzahnrads angeordnet ist. Dementsprechend weist der Außenring eine Innenverzahnung und weisen der Innenring und die Zwischenringe eine Außenverzahnung auf. Hierdurch kann eine Übersetzung der Drehung und Kraft und somit ein schnelleres oder leichteres Öffnen und Schließen der Verschraubung realisiert werden. Das Kuppeln bzw. Entkuppeln der Betätigungselemente-Paare ist hierdurch deutlich schneller möglich.

Nachfolgend wird die Erfindung anhand bevorzugter Ausführungsformen unter Bezugnahme auf die anliegenden Zeichnungen näher erläutert.

Es zeigen:
- Figur 1: eine schematische, perspektivische Ansicht eines chirurgischen Manipulationsinstruments,
- Figur 2: eine schematische, perspektivische Seitenansicht der Antriebseinrichtung,
- Figur 3: schematische Ansichten der ersten bevorzugten Ausführungsform der Erfindung in der die Betätigungseinrichtungen über die Kuppeleinrichtung noch nicht miteinander verbunden sind,
- Figur 4: schematische Ansichten einer ersten bevorzugten Ausführungsform der Erfindung in der die Betätigungseinrichtungen über die Kuppeleinrichtung miteinander verbunden sind,
- Figur 5: eine schematische Seitenansicht einer zweiten bevorzugten Ausführungsform der Erfindung, wobei die Betätigungseinrichtungen noch nicht über die Kupplungseinrichtungen miteinander verbunden sind
- Figur 6: eine schematische, perspektivische Ansicht der in Figur 5 dargestellten Ausführungsform, wobei die Betätigungseinrichtungen über die Kuppeleinrichtungen miteinander verbunden sind,
- Figur 7: eine schematische perspektivische Ansicht einer weiteren Kupplungseinrichtung für chirurgische Manipulationsinstrumente und
- Figur 8: eine schematische Querschnittansicht der in Figur 7 dargestellten Kupplungseinrichtung.

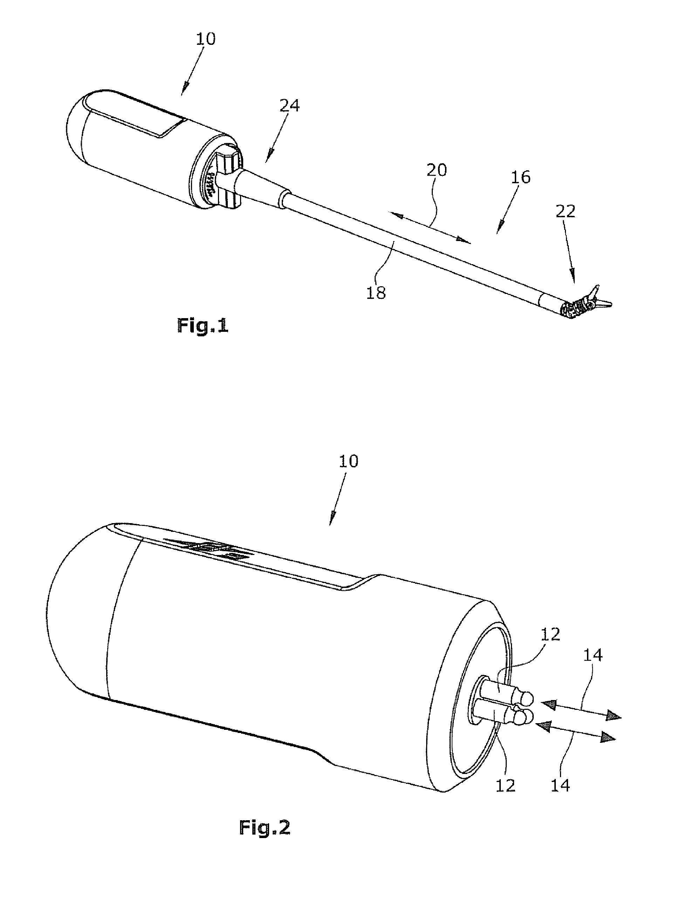

Ein erfindungsgemäßes chirurgisches Manipulationsinstrument, das insbesondere für die minimal invasive Chirurgie geeignet ist weist eine extrakorporale Antriebseinrichtung 10 auf. Diese kann insbesondere einen oder mehrere Elektromotoren und Getriebe aufweisen, wobei hierdurch Betätigungselemente 12 (Figur 2) in axiale Richtung (Pfeile 14) verschoben werden.

Diese ersten Betätigungselemente 12 sind im dargestellten Ausführungsbeispiel als Stangen ausgebildet Es kann sich jedoch auch um Seile handeln, über die dann ausschließlich Zugkräfte übertragen werden.

Ferner weist das chirurgische Manipulationsinstrument eine Manipulatoreinrichtung 16 (Figur 1) auf. Innerhalb eines rohrförmigen Schafts 18 sind wiederum stabförmige zweite Betätigungselemente angeordnet. Diese sind in Längsrichtung 20 verschiebbar. Die zweiten Betätigungselemente sind mit einem Endeffektor 22 verbunden. Durch Verschieben der zweiten Betätigungselemente erfolgt somit ein Betätigen des Endeffektors. Bei den zweiten Betätigungselementen kann es sich wiederum um stabförmige Betätigungselemente aber auch um Betätigungsseile handeln.

Zur Verbindung der ersten Betätigungselemente 12 und der innerhalb des rohrförmigen Schafts angeordneten zweiten Betätigungselementen ist eine Kupplungseinrichtung 24 vorgesehen.

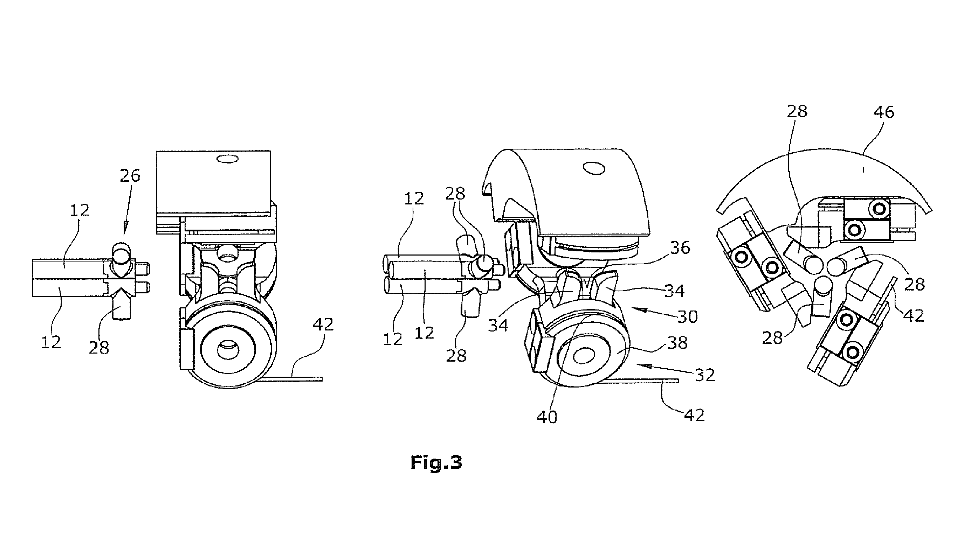

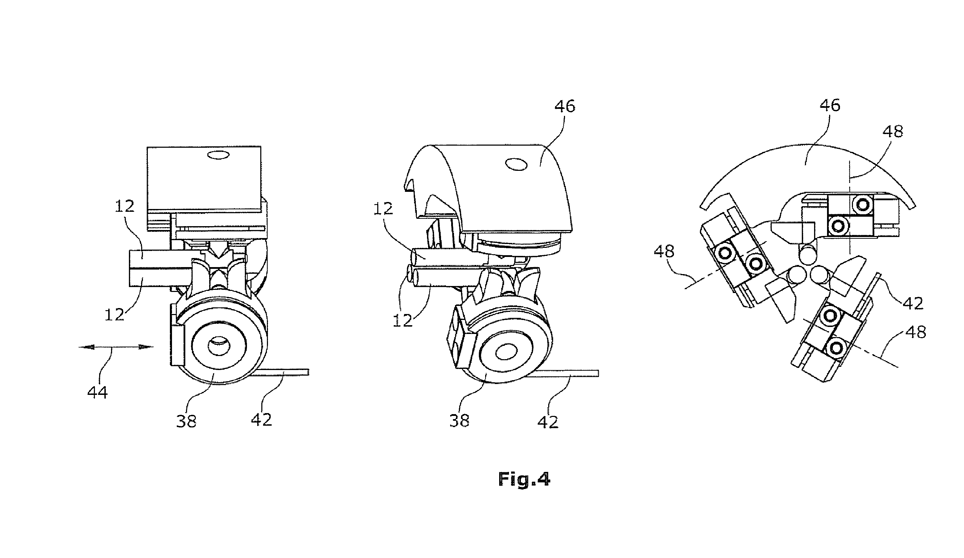

Bei einer ersten bevorzugten Ausführungsform (Fig. 3 und 4) sind die in Figur 2 angedeuteten ersten Betätigungselemente 12 als drei parallel zueinander verlaufende Stäbe ausgebildet. An den Enden der Stäbe sind erste Kupplungselemente 26 vorgesehen. Die Kupplungselemente 26 weisen jeweils einen stiftförmigen Ansatz 28 auf. Der stiftförmige Ansatz 28 ist im dargestellten Ausführungsbeispiel zylindrisch ausgebildet und ragt in einem Winkel von 90 Grad zur Längsachse des ersten Betätigungselements 12 nach außen. Wie aus der Draufsicht (Fig. 3 rechts) ersichtlich ist, sind die drei zylindrischen Ansätze 28 gleichmäßig verteilt angeordnet und weisen somit einen Winkel von 120 Grad zueinander auf.

Zweite Kupplungselemente 30 weisen im dargestellten Ausführungsbeispiel einen Schwenkarm 32 auf. Der Schwenkarm 32 weist zwei zueinander parallel verlaufende Armteile 34 auf, so dass eine gabelförmige Ausnehmung 36 ausgebildet ist. In der gabelförmigen Ausnehmung 36 wird, wie nachstehend beschrieben, der stiftförmige Ansatz 28 der ersten Kupplungselemente 26 aufgenommen.

Der Schwenkarm 32 ist fest mit einem scheibenförmigen Element 38 des Zwischenelements 32 verbunden.

Im dargestellten Ausführungsbeispiel weist das Scheibenelement 38 eine Rille 40 auf, in der ein Seilzug 42 angeordnet ist. Aus Gründen der Übersichtlichkeit ist dieser nur an einer der Scheiben dargestellt. Durch Drehen des Scheibenelements 38 in eine entsprechende Richtung erfolgt somit ein Ziehen an dem Seilzug 42. Der Seilzug 42 entspricht der zweiten Betätigungseinrichtung oder ist zumindest mit dieser verbunden.

Zum Schließen der Kupplungseinrichtung erfolgt in Draufsicht (Fig. 3 rechts) ein gemeinsames Drehen aller drei ersten Betätigungselemente 12 im Uhrzeigersinn, so dass die Ansätze 28 der einzelnen Kupplungselemente 26 in die entsprechenden gabelförmigen Ausnehmungen eingreifen.

Nach dem Kuppeln der Kupplungselemente 26, 30 können von den ersten Betätigungseinrichtungen 12 durch Verschieben dieser in Richtung eines Pfeils 44 (Fig. 4) Kräfte auf die zweiten Betätigungseinrichtungen 42 übertragen werden. Da es sich im dargestellten Ausführungsbeispiel bei der zweiten Betätigungseinrichtung 42 um ein Seil handelt, können hierbei nur Zugkräfte übertragen werden. Erfolgt somit beispielsweise ein Verschieben des ersten Betätigungselements 12 in Figur 4 nach rechts, so erfolgt ein Drehen des Scheibenelements 38 im Uhrzeigersinn und somit ein Ziehen an der einzigen in Figur 4 dargestellten zweiten Betätigungseinrichtung 42.

Die im dargestellten Ausführungsbeispiel drei schwenkbaren Zwischenelemente 32 sind in einem nur teilweise dargestellten Gehäuse 46 um Schwenkachsen 48 schwenkbar gehalten.

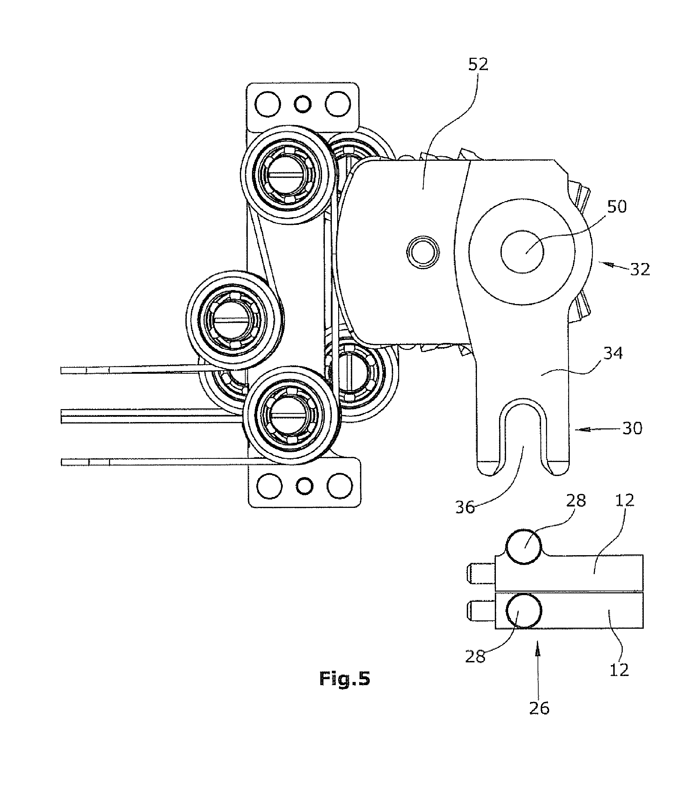

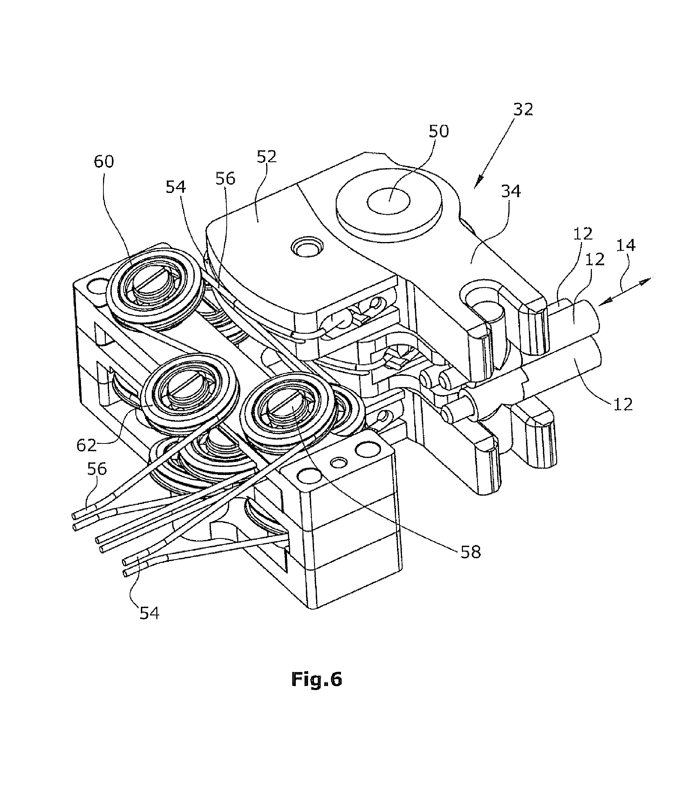

Bei einer weiteren bevorzugten Ausführungsform der Erfindung (Fig. 5 und 6) sind identische und ähnliche Bauteile mit denselben Bezugszeichen gekennzeichnet.

Die ersten Betätigungselemente 12 sind wiederum stabförmig ausgebildet und weisen Ansätze 28 auf, wobei diese aufgrund der Anordnung der zweiten Kupplungselemente 30 eine räumlich andere Anordnung haben. Die gabelförmigen Ausnehmungen 36 der in dieser Ausführungsform drei Kupplungselemente 30 sind in dieser Ausführungsform in Figur 5 hintereinander angeordnet. Entsprechend weist das in Figur 5 untere erste Betätigungselement 12 einen Ansatz 28 auf, der aus der Zeichenebene heraussteht. Hinter diesem Betätigungselement 12 ist ein weiteres erstes Betätigungselement 12 angeordnet, dessen Ansatz 28 in die entgegengesetzte Richtung weist. Das dritte in Figur 5 obere Betätigungselement 12 weist einen sich in beide Richtungen erstreckenden Ansatz 28 auf, der jedoch eine geringere Länge hat. Dieser Ansatz 28 greift in die in Figur 6 mittlere Gabelöffnung 36.

Im eingekuppelten Zustand bewirkt ein Verschieben der ersten Betätigungselemente 12 in Längsrichtung 14 ein Verschwenken des Schwenkarms 32 um eine Schwenkachse 50. Im dargestellten Ausführungsbeispiel sind die Schwenkachsen 50 sämtlicher Zwischenelemente 32 koaxial zueinander.

In dem in Figur 5 und 6 dargestellten Ausführungsbeispiel ist das Zwischenelement 32 jeweils starr mit einem Verbindungsarm 52 verbunden. Der Verbindungsarm 52 der Zwischenelemente 32 weist im dargestellten Ausführungsbeispiel einen rechten Winkel zu dem Schwenkarm 32 auf. Der Verbindungsarm 52 ist sodann mit zwei Seilzügen verbunden. Dies ist insbesondere bei dem in Figur 6 oberen Verbindungsarm 52 ersichtlich, der mit den beiden Seilzügen 54, 56 verbunden ist. Die beiden Seilzüge 54, 56 sind auf unterschiedlichen Seiten mit dem Verbindungsarm 52 verbunden. Hierdurch erfolgt je nach Drehrichtung des Zwischenelements 32 ein Ziehen an dem einen oder dem anderen Seilzug 54 bzw. 56. Bei einem Verschwenken des Zwischenelements 32 in Figur 6 im Uhrzeigersinn erfolgt ein Ziehen des Seilzugs 54. Dieser ist über eine Umlenkrollen 58 gelenkt und mit einem zweiten Betätigungselement verbunden oder geht in das zweite Betätigungselement über. Beim Schwenken des Zwischenelements 32 gegen den Uhrzeigersinn (Fig. 6) erfolgt ein Ziehen an dem Seilzug 56. Dieser ist über zwei Umlenkrollen 60, 62 umgelenkt und verläuft sodann parallel zu dem Seilzug 54. Je nach Verschieberichtung des ersten Betätigungselements 12 in axialer Richtung 14 erfolgt somit ein Ziehen an dem Seilzug 54 oder 56. Bei dieser Ausführungsform können somit auch beim Vorsehen von Seilzügen beide Bewegungsrichtungen der ersten Betätigungselemente genutzt werden. In dem dargestellten Ausführungsbeispiel sind die beiden anderen Zwischenelemente ebenfalls jeweils mit zwei Seilzügen verbunden, die ebenfalls wiederum über Umlenkrollen geführt sind. Insofern sind sechs Seilzüge vorgesehen, über die Zugkräfte unmittelbar oder unter Zwischenhaltung zweiter Betätigungselemente auf Elemente des Endeffektors 22 (Fig. 1) übertragen werden können.

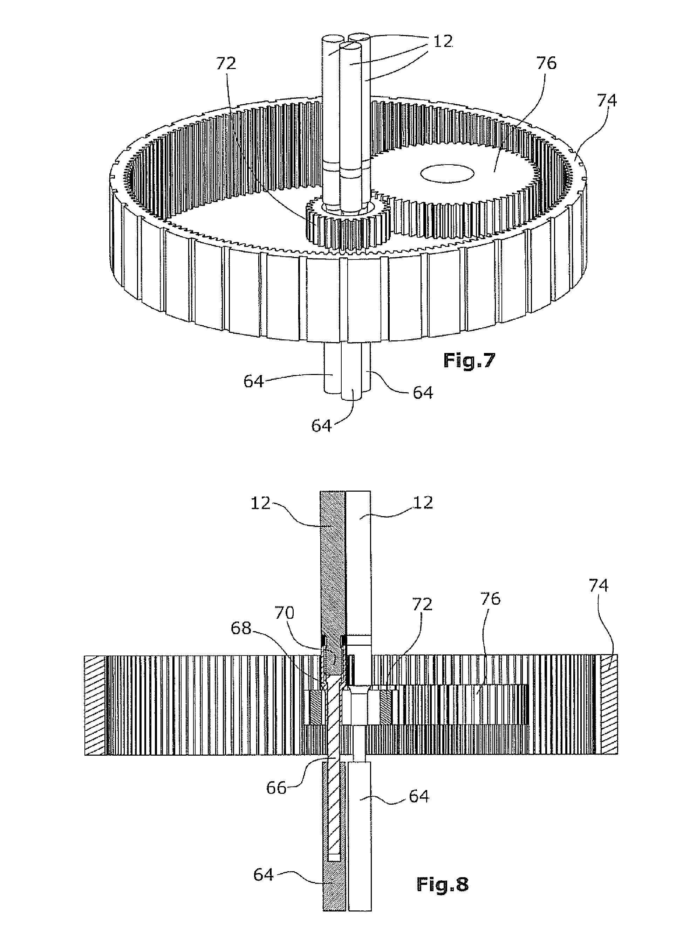

Bei einer weiteren Ausführungsform einer Kupplungseinrichtung für ein chirurgisches Manipulationsinstrument sind ebenfalls Betätigungselemente-Paare vorgesehen, wobei die ersten Betätigungselemente 12 wiederum stabförmig sind. Die zweiten Betätigungselemente 64 sind im dargestellten Ausführungsbeispiel ebenfalls stabförmig, Entsprechend der vorstehend beschriebenen Ausführungsform sind die ersten Betätigungselemente 12 mit der in den Figuren 1 und 2 dargestellten Antriebseinrichtung 10 und die zweiten Betätigungselemente 64 mit dem Endeffektor 22 verbunden. Bei der in den Figuren 7 und 8 dargestellten Ausführungsform handelt es sich um eine selbstständige Erfindung,

Das Verbinden der Betätigungselemente-Paare 12-64 erfolgt in diesem Ausführungsbeispiel durch Verschraubung. Hierzu weisen die zweiten Betätigungselemente 64 einen stiftförmigen Ansatz 66 auf, der fest mit dem zweiten Betätigungselement verbunden ist. Der stiftförmige Ansatz 66 ist mit einer Verbindungshülse 68 verbunden, die ein Innengewinde aufweist und rotatorisch beweglich ist. Die ersten Betätigungselemente 12 weisen an ihrem in Richtung des zweiten Betätigungselements 64 weisenden Ende einen Zapfen 70 mit Außengewinde auf. Durch Drehen der Verbindungshülsen 68 um ihre Längsachse ist somit ein Verschrauben, d.h. ein Kuppeln der Betätigungselemente-Paare 12-64 möglich, ohne diese zu rotieren.

Um die im dargestellten Ausführungsbeispiel drei Betätigungselemente-Paare 12-64 gleichzeitig miteinander zu verbinden, ist ein Innenring 72 entsprechend einem Sonnenrad vorgesehen, der im dargestellten Ausführungsbeispiel die außen verzahnten Verbindungshülsen 68 umgibt und in Kupplungsposition in die Zähne der Verbindungshülsen eingreift. Das Sonnenrad 72 weist ein Innengewinde auf, das mit einem an der Außenseite der Hülsen 68 vorgesehenen Gewinde zusammenwirkt. Durch ein Drehen des Innenrings 72 werden somit die Betätigungselemente 12, 64 miteinander verschraubt. Um ein möglichst schnelles Öffnen und Schließen der Schraubverbindung zu ermöglichen, ist ein Außenring 74 wie ein Handrad vorgesehen, der das Sonnenrad 72 umgibt. Zwischen den beiden Ringen 72, 74 ist mindestens ein, vorzugsweise drei Übertragungszahnräder 76 wie Planetenräder vorgesehen, das in eine an der Innenseite des Handrads 74 sowie in eine an der Außenseite des Sonnenrads 72 vorgesehene Verzahnung eingreift. Um Festigungstoleranzen auszugleichen, ist zwischen dem Betätigungselement 12 und der Verbindungshülse 68 ein elastisches Ausgleichelement angefügt, gegen welches die Verbindungshülsen 68 durch Drehen des Sonnenrads 72 gespannt werden.

## Patentansprüche

1. Chirurgisches Manipulationsinstrument, insbesondere für die minimal invasive Chirurgie, mit
einer extrakorporalen Antriebseinrichtung (10), mit mehreren axial verschiebbaren ersten Betätigungselementen (12),
einem teilweise intrakorporalen Manipulatorteil (16), mit mehreren axial verschiebbaren zweiten Betätigungselementen (42, 54, 56) zum Betätigen eines Endeffektors (22) und
einer Kupplungseinrichtung (24) zum lösbaren Verbinden von Betätigungselementen-Paaren, wobei die Kupplungseinrichtung (24) ein mit dem ersten Betätigungselement (12) des jeweiligen Paares verbundenes erstes Kupplungselement (26) und ein mit dem zweiten Betätigungselement (42, 54, 56) des jeweiligen Paares verbundenes zweites Kupplungselement (30) aufweist,
**dadurch gekennzeichnet, dass**
zwischen dem zweiten Kupplungselement (30) und dem zweiten Betätigungselement (42, 54, 56) ein schwenkbares Zwischenelement (32) vorgesehen ist, durch das die axiale Bewegung des ersten Betätigungselements (12) in eine Schwenkbewegung überführt wird, die von dem Zwischenelement (32) in eine axiale Bewegung des zweiten Betätigungselements (42, 54, 56) überführt wird.

2. Chirurgisches Manipulationsinstrument nach Anspruch 1, **dadurch gekennzeichnet, dass** die zweiten Kupplungselemente (30) einen Schwenkarm (34) aufweisen, der mit dem Zwischenelement (32) insbesondere starr verbunden ist.

3. Chirurgisches Manipulationsinstrument nach Anspruch 2, **dadurch gekennzeichnet, dass** der Schwenkarm (34) eine gabelförmige Ausnehmung (36) aufweist, in die ein Ansatz (28) des ersten Kupplungselements (26) eingreift.

4. Chirurgisches Manipulationsinstrument nach einem der Ansprüche 1-3, **dadurch gekennzeichnet, dass** die Schwenkachse (48) des Zwischenelements (32) im Wesentlichen senkrecht zu der jeweiligen Bewegungsrichtung (14) des zugehörigen ersten Betätigungselements (12) ist.

5. Chirurgisches Manipulationsinstrument nach einem der Ansprüche 1-4, **dadurch gekennzeichnet, dass** Zwischenelement (32) ein zumindest teilkreisförmiges Scheibenelement (38) aufweist, an dessen Außenumfang ein elastisches insbesondere seilförmiges Verbindungselement (42, 54, 56) angeordnet ist, das mit dem zweiten Betätigungselement (42, 54, 56) verbunden ist.

6. Chirurgisches Manipulationsinstrument nach einem der Ansprüche 1-4, **dadurch gekennzeichnet, dass** das Zwischenelement (32) einen insbesondere starr mit dem Schwenkarm (34) verbundenen Verbindungsarm (52) aufweist.

7. Chirurgisches Manipulationsinstrument nach Anspruch 6, **dadurch gekennzeichnet, dass** der Verbindungsarm (52) über ein elastisches insbesondere seilförmiges Verbindungselement (54, 56) mit dem zweiten Betätigungselement (54, 56) verbunden ist.

8. Chirurgisches Manipulationsinstrument nach Anspruch 7, **dadurch gekennzeichnet, dass** das Verbindungselement (54, 56) über mindestens eine Umlenkrolle (58, 60, 62) geführt ist, die insbesondere einen Teil des Zwischenelements ausbildet.

9. Chirurgisches Manipulationsinstrument nach einem der Ansprüche 6-8, **dadurch gekennzeichnet, dass** der Verbindungsarm (52) mit zwei elastischen insbesondere seilförmigen Verbindungselementen (54, 56) verbunden ist, so dass in Abhängigkeit der Schwenkrichtung über das eine oder das andere Verbindungselement (54, 56) Zugkräfte übertragen werden.

## Claims

1. A surgical manipulation instrument, in particular for minimally invasive surgery, comprising
an extra-corporeal drive device (10) with a plurality of axially displaceable first actuation elements (12),
a partly intra-corporeal manipulator part (16) with a plurality of axially displaceable second actuation devices (42, 54, 56) for the actuation of an end effector (22), and
a coupling device (24) for the detachable coupling of actuation element pairs, wherein the coupling device (24) comprises a first coupling element (26) connected with the first actuation element (12) of the respective pair and a second coupling element (30) connected with the second actuation element (42, 54, 56) of the respective pair,
**characterized in that**
a pivotable intermediate element (32) is provided between the second coupling element (30) and the second actuation element (42, 54, 56), by which intermediate element the axial movement of the first actuation element (12) is translated into a pivot movement which is translated by the intermediate element (32) into an axial movement of the second actuation element (42, 54, 56).

2. The surgical manipulation instrument of claim 1, **characterized in that** the second coupling elements (30) comprise a pivot arm (34) which is in particular rigidly connected with the intermediate element (32).

3. The surgical manipulation instrument of claim 2, **characterized in that** the pivot arm (34) has a bifurcated recess (36) into which engages a projection (28) of the first coupling element (26).

4. The surgical manipulation instrument of one of claims 1-3, **characterized in that** the pivot axis (48) of the intermediate element (32) is substantially perpendicular to the respective direction of movement (14) of the associated first actuation element (12).

5. The surgical manipulation instrument of one of claims 1-4, **characterized in that** the intermediate element (32) comprises an at least segment-shaped disc element (38) which has its outer circumference provided with an elastic, in particular cable-like, connecting element (42, 54, 56) connected with the second actuation element (42, 54, 56).

6. The surgical manipulation instrument of one of claims 1-4, **characterized in that** the intermediate element (32) comprises a connecting arm (52) which is connected with the pivot arm (34) in particular in a rigid manner.

7. The surgical manipulation instrument of claim 6, **characterized in that** the connecting arm (52) is connected with the second actuation element (54, 56) through an elastic, in particular cable-like connecting element (54, 56).

8. The surgical manipulation instrument of claim 7, **characterized in that** the connecting element (54, 56) is guided over at least one guide roller (58, 60, 62) which in particular forms a part of the intermediate element.

9. The surgical manipulation instrument of one of claims 6-8, **characterized in that** the connecting arm (52) is connected with two elastic, in particular cable-like connecting elements (54, 56) so that pulling forces are transmitted via the one or the other connecting element (54, 56), depending on the pivoting direction.

## Revendications

1. Instrument de manipulation à usage chirurgical, en particulier pour la chirurgie invasive minimale, comprenant :
- un dispositif d'entraînement extracorporel (10), comprenant plusieurs premiers éléments d'actionnement (12), pouvant être déplacés axialement,
- une partie manipulateur en partie intracorporel (16), comprenant plusieurs seconds éléments d'actionnement (42, 54, 56) pouvant être déplacés axialement afin d'actionner un effecteur terminal (22) et
- un dispositif d'accouplement (24) destiné à relier de manière amovible des paires d'éléments d'actionnement, le dispositif d'accouplement (24) présentant un premier élément d'accouplement (26) relié au premier élément d'actionnement (12) de la paire respective et un second élément d'accouplement (30) relié au second élément d'actionnement (42, 54, 56) de la paire respective,
**caractérisé en ce que**
entre le second élément d'accouplement (30) et le second élément d'actionnement (42, 54, 56) est prévu un élément intermédiaire (32) pivotant par l'intermédiaire duquel le mouvement axial du premier élément d'actionnement (12) est transformé en un mouvement de pivotement, qui est transformé par l'élément intermédiaire (32) en un mouvement axial du second élément d'actionnement (42, 54, 56).

2. Instrument de manipulation à usage chirurgical selon la revendication 1, **caractérisé en ce que** les seconds éléments d'accouplement (30) présentent un bras pivotant (34), qui est relié de manière particulièrement rigide avec l'élément intermédiaire (32).

3. Instrument de manipulation à usage chirurgical selon la revendication 2, **caractérisé en ce que** le bras pivotant (34) présente une cavité (36) en forme de fourche, dans laquelle vient s'engager un appendice (28) du premier élément d'accouplement (26).

4. Instrument de manipulation à usage chirurgical selon l'une des revendications 1 à 3, **caractérisé en ce que** l'axe de pivotement (48) de l'élément intermédiaire (32) est sensiblement perpendiculaire à la direction de déplacement (14) respective du premier élément d'actionnement (12) associé.

5. Instrument de manipulation à usage chirurgical selon l'une des revendications 1 à 4, **caractérisé en ce que** l'élément intermédiaire (32) présente un élément formant un disque (38), de forme tout au moins en partie circulaire, à la périphérie externe duquel est agencé un élément de raccordement (42, 54, 56) élastique et particulièrement en forme de câble, qui est relié au second élément d'actionnement (42, 54, 56).

6. Instrument de manipulation à usage chirurgical selon l'une des revendications 1 à 4, **caractérisé en ce que** l'élément intermédiaire (32) présente un bras de raccordement (52) relié de manière particulièrement rigide au bras pivotant (34).

7. Instrument de manipulation à usage chirurgical selon la revendication 6, **caractérisé en ce que** le bras de raccordement (52) est relié au second élément d'actionnement (54, 56) par l'intermédiaire d'un élément de raccordement (54, 56) élastique et particulièrement en forme de câble.

8. Instrument de manipulation à usage chirurgical selon la revendication 7, **caractérisé en ce que** l'élément de raccordement (54, 56) est guidé par l'intermédiaire d'au moins une poulie de renvoi (58, 60, 62) qui forme en particulier une partie de l'élément intermédiaire.

9. Instrument de manipulation à usage chirurgical selon l'une des revendications 6 à 8, **caractérisé en ce que** le bras de raccordement (52) est relié à deux éléments de raccordement (54, 56) élastiques et particulièrement en forme de câble, de telle sorte que des forces de traction sont transmises sur l'un ou l'autre élément de raccordement (54, 56) en fonction du sens de pivotement.
